Europäisches Patentamt

European Patent Office

Office européen des brevets

(19)

(11) Publication number: **0 206 691**
A2

(12) **EUROPEAN PATENT APPLICATION**

(21) Application number: **86304570.4**

(22) Date of filing: **13.06.86**

(51) Int. Cl.⁴: **C 12 N 5/00**

(30) Priority: **17.06.85 JP 132809/85**

(43) Date of publication of application: **30.12.86**
**Bulletin 86/52**

(84) Designated Contracting States: **CH DE FR GB IT LI NL SE**

(71) Applicant: **Nippon Paint Co., Ltd., 2-1-2, Oyodokita Oyodo-ku, Osaka-shi Osaka-fu (JP)**

(72) Inventor: **Yamamoto, Yoshikazu, 24-1-402, Ikedakitamachi, Neyagawa-shi Osaka-fu (JP)**
Inventor: **Mizuguchi, Ryuzo, C-410, No. 8, Yawatakitaura, Yawata-shi Kyoto-fu (JP)**

(74) Representative: **Perry, Robert Edward et al, GILL JENNINGS & EVERY 53-64 Chancery Lane, London WC2A 1HN (GB)**

(54) Production of deuterium-containing chemical substances.

(57) By using a medium wherein not less than 85% of the total hydrogen atoms are deuterium, plant cells are cultivated and deuterated chemical substances can be recovered from the culture.

## PRODUCTION OF DEUTERIUM-CONTAINING CHEMICAL SUBSTANCES

The present invention relates to production of deuterium-containing (i.e. deuterated) chemical substances. More particularly, it relates to deuterium-containing media for plant cell culture, plant cell cultivation with said media, plant cell culture products from culture mixtures after said cultivation, recovery of deuterium-containing chemical substances from said culture products and deuterium-containing chemical substances thus recovered.

Deuterium is used widely as a stable nuclear species in production of labeled compounds, and there are known a number of deuterated organic and inorganic substances. These deuterated chemical substances are usually produced by chemical syntheses, and production of deuterated chemical substacnes by chemical syntheses becomes difficult with enhancement of complexity of their molecular structures and increase of the deuterium contents therein. On the other hand, the demands to chemical substances having high deuterium contents as reagents or tracers for research on the biotic metabolism and the biological activity are increased in recent years.

Aiming at efficient production of chemical substances having high deuterium contents, an extensive study has been made, and in the course of such study, attempt has been made to attain said aim by utilization of plant cells having a capability of producing chemical substances. Since plant cells are considered to have no capability of differentiating usual hydrogen atoms from heavy hydrogen atoms (i.e. deuterium), it may be necessary to keep high concentrations of deuterium in media wherein plant cells are cultivated for production of chemical substances having high deuterium contents.

Hitherto, cultivation of some kinds of microorganisms including bacteria and fungi in deuterium-containing media was made, and growth of those microrganisms was recognized to be successful in some cases and not successful in some other cases. But, there is no report on cultivation of plant cells other than microorganisms in deuterium-containing media. Therefore, it was not predictable whether plant cells can grow in media having high deuterium concentrations. It was also not predictable whether higher plant cells can well grow in media having high deuterium concentrations to an extent sufficient to recover the produced deuterium-containing chemical substances from said media.

It has now been found that plant cells, particularly higher plant cells, can in general well grow in deuterium-containing media, although suppression on the growth may be somewhat observed. Particularly when cultivation is effected with gradual increase of the deuterium concentration in the media, the plant cells are provided with adaptability, and their growth proceeds smoothly. Chemical substances having higher deuterium contents can be produced with the progress of the growth. This invention is based on the above findings.

According to the present invention, there is

- 3 -

0206691

provided a process for cultivation of plant cells to produce chemical substances having high deuterium contents, characterized in that the cultivation is effected in a nutrient medium wherein not less than 85 % of the total hydrogen atoms are deuterium.

This invention will be hereinafter explained more in detail taking production of deuterated tocopherol by cultivation of Chlorella ellipsoidea as a typical example. Needless to say, this invention should not be understood to be limited to this typical example only. In other words, this invention may be likewise applied to cultivation of various plant cells other than chlorella cells for production of various chemical substances. Particulrly when it is applied to cultivation of plant cells which can grow in a photoautotrophic system, chemical substances wherein all the hydrogen atoms are deuterium are obtainable. In this case, the characteristic effect of the invention can be the most well recognized.

For production of chemical substances having high deuterium contents according to the process of the invention, a plant cell having a capability of a desired chemical substance is added to a nutrient medium containing deuterium under sterilized conditions. The nutrient medium may be anyone appropriately chosen from conventional liquid and solid media depending upon the purpose, insofar as deuterium is contained and not less than 85 % of the total hydrogen atoms are deuterium. Namely, the basic composition of the nutrient medium may comprise water, inorganic salts (e.g.

- 4 -

0206691

sodium salts, potassium salts, calcium salts, magnesium salts, ammonium salts, phosphates, sulfates, nitrates), carbon sources (e.g. sucrose, glucose, fructose, maltose, raffinose, starch, dextrin), growth regulators (e.g. auxin, cytokinin, kinetin, benzyladenine), organic trace substances (e.g. thiamine, nicotinic acid, pyridoxin, p-aminobenzoic acid, folic acid, choline, myoinositol, glycine), natural extracts (e.g. casein hydrolyzate, yeast extract, malt extract, coconut milk), agar,·etc. In order to have deuterium included, heavy water (i.e. deuterated water) is usually employed instead of normal water. Other deuterated substances utilizable in the metabolism of the plant cell as used may be also employed.

In case of chlorella, for instance, there may be employed Kanazawa medium as conventionally used for the cultivation of chlorella, in which, however, water is substituted with heavy water (having a deuterium content of not less than 85 %). Preferably, all the hydrogen atoms in all the components in the nutrient medium may be replaced by deuterium, whereby chemical substances in which all the hydrogen atoms are deuterium are obtainable. In case of algal cells other than chlorella, there may be used Borbox medium, Bristol medium, Ditmer medium, etc., in which water is replaced by heavy water. In case of higher plant cells, there may be used White medium, Linsmaier-Skoog medium, etc. in which water is substituted with heavy water.

Prior to addition of the plant cell, the nutrient medium is usually subjected to treatment for elimination of contaminating microorganisms therefrom. For this purpose, there may be adopted autoclave method, steam sterilization method, etc. When, however, the deuterium content in the nutrient medium is high, the elimination is preferably attained by filtration or irradiation with gamma-rays.

The nutrient medium to which the plant cell was added is then cultivated at an appropriate temperature over a certain period of time while maintaining a suitable pH depending upon the kind and property of the plant cell. In case of chlorella, for instance, the cultivation is usually carried out at a temperature around 25°C for about 2 weeks. The cultivation may be effected by any per se conventional procedure such as shaking culture, agitation culture, stationary culture, etc., and an appropriate one may be chosen therefrom taking the kind and property of the plant cell into consideration. The cultivation period may be also appropriately decided depending on the kind of the plant cell and the production efficiency of the objective chemical substance. When the plant cell is photoautotrophic like chlorella, light irradiation is needed for growth, and usually a light intensity of not less than 1,000 lux, particularly of not less than 5,000 lux, is desirable. When desired, air or oxygen may be introduced into the nutrient medium. In case of using a photoautotrophic one, aeration enriched with carbon dioxide is particularly preferred for promotion of the growth.

From the thus obtained culture mixture which comprises the grown plant cells and the nutrient medium, the

- 6 -

0206691

deuterium-containing chemical substances are recovered. Separation of the plant cells from the culture mixture may be effected by a per se conventional separation procedure such as filtration, centrifugation and/or extraction. The separated plant cells or nutrient medium may be obtained in a dry state by lyophilization and/or drying with hot air. Separation of the deuterium-containing substances from the wet or dried product of the plant cells or nutrient medium may be attained by the separation procedure applicable to the non-deuterium containing substances corresponding to the deuterium-containing substances. For instance, non-polar and oil-soluble substances such as sterols and fatty acids can be effectively recovered by extraction with organic solvents (e.g. methanol, acetone, ether, benzene, ethyl acetate) is effective. Further, for instance, water-soluble substances such as glycosides and polysaccharides can be effectively recovered by extraction with aqueous solvents such as hot water and aqueous alcohols. When chlorella cells are cultivated for production of deuterated tocopherols, the culture mixture is filtered to separate into the nutrient medium as a liquid and the chlorella cells as a solid, and the chlorella cells are centrifuged, washed with water and dried to obtain a dried chlorella cell product. This dried product is extracted with an organic solvent such as acetone or methanol, and the resulting extract is distilled to remove the organic solvent. The residue is subjected to column chromatography to obtain deuterated tocopherol.

Confirmation of the production of deuterated substances is usually made by mass spetrometry. When, for instance, the deuterated substance is tocopherol, the parent peak of deuterated tocopherol may be distributed over the range from the one due to non-deuterated tocopherol ($C_{29}H_{50}O_2$) having a molecular weight of 430 to the one due to perfectly deuterated tocophenol ($C_{29}D_{50}O_2$) having a molecular weight of 480 unless the deuterium content of the nutrient medium is 100 %. Since non-deuterated tocopherol produces peaks of p+1 and p+2 respectively at m/z 431 and at m/z 432, observation of peaks having higher intensities at m/z 431 and 432 or of any peak at m/z 433 or more means the presence of deuterated substances. When any peak is not observed between m/z 430 and 479 and only the peaks at m/z 480, 481 and 482 are observed, the product may be confirmed to be perfectly deutrated.

Confirmation of deuterated substances in the extract or the plant cell dried product without their isolation may be effected by isolating any typical component from said extract or said plant cell dried product and examining whether the typical component is deuterated or not.

Practical and presently preferred embodiments of the invention are illustratively shown in the following Examples.

Example 1

| Material | Amount |
| --- | --- |
| Glucose | 1 g |
| L-Asparagine | 0.2 g |
| Potassium dihydrogen phosphate | 0.1 g |
| Magnesium sulfate | 0.05 g |
| Iron nitrate | 0.02 mg |
| Zinc sulfate | 0.02 mg |
| Thiamine hydrochloride | 10 μg |
| Biotin | 0.5 μg |

The above materials were dissolved in heavy water (100 ml) and adjusted to pH 5.8, followed by filtration through a membrane filter of 0.22 mμ to eliminate contaminating bacteria. In the resultant medium (i.e. Lilly-Bernett medium) having a deuterium content of about 99 %, a cultured tissue (1 g) comprising algal cells and fungous cells induced from thallus of Usnea rubescens (Usneaceae, Lecanorales) collected at Kyoto-shi, Japan was cultivated at 20°C over a period of 2 months. The grown cultivated tissue (5 g) was lyophilized for 10 hours. The obtained dried product (about 1 g) was dipped in n-hexane for 24 hours and filtered. The filtrate was concentrated under reduced pressure to remove the solvent. The residue (about 20 mg) was dissolved in a small amount of ether and spotted on a thin layer of silica gel, followed by development with a mixture of n-hexane, ether and formic acid. After drying in the air, the spot having the same Rf value as the standard sample of usnic acid and giving fluorescence on irradiation

- 9 -

0206691

with ultraviolet rays was scraped off and subjected to measurement with mass spectrography. In case of the standard sample of usnic acid ($C_{18}H_{16}O_7$), a parent peak at m/z 344 and peaks of p+1 and p+2 are observed. In comparison of the mass spectrum of the specimen obtained in this Example with that of the standard sample, the presence of a parent peak at m/z 344 or more is observed in the former. As the result, the presence of deuterated usnic acid in the specimen was confirmed.

Example 2

| Material | Amount |
| --- | --- |
| Sucrose | 3 g |
| Ammonium nitrate | 0.165 g |
| Potassium nitrate | 0.19 g |
| Calcium chloride | 44 mg |
| Magnesium sulfate | 37 mg |
| Potassium dihydrogen phosphate | 17 mg |
| Boric acid | 0.62 mg |
| Manganese sulfate | 2.23 mg |
| Zinc sulfate | 0.86 mg |
| Potassium iodide | 0.083 mg |
| Sodium molybdate | 0.025 mg |
| Cobalt chloride | 2.5 μg |
| Cupric sulfate | 2.5 μg |
| Sodium ethylenediaminetetraacetate | 3.73 mg |
| Ferrous chloride | 2.78 mg |
| Mio-innositol | 10 mg |
| Glycine | 0.2 mg |

| | |
|---|---|
| Pyridoxin hydrochloride | 0.05 mg |
| Nicotinic acid | 0.05 mg |
| Thiamine hydrochloride | 0.01 mg |
| 2,4-Dichlorophenoxyacetic acid | $10^{-7}M$ |
| Kainetin | $10^{-6}M$ |

The above materials were dissolved in heavy water (100 ml) and adjusted to pH 6.0, followed by filtration through a membrane filter of 0.22 mµ to eliminate contaminating bacteria. In the resultant medium (i.e. Murashige-Skooge medium) having a deuterium content of about 98 %, a cultured tissue (1 g) induced from petiole of Hydrocotyle maritima (Umbelliferae, Hydrocotyle) collected at Kyoto-shi, Japan was cultivated at 25°C in a dark place with rotatory shaking (140 rpm) over a period of 4 weeks. The culture mixture was filtered to collect the cells (6 g), which were immersed in benzene (100 ml). The nutrient medium (100 ml) was shaken with benzene (100 ml), and the benzene extract was separated and combined with the benzene extract of the cells. The combined extract was distilled under reduced pressure to remove the solvent. The residue (about 8 mg) was dissolved in a small amount of benzene and spotted on a thin layer of silica gel, followed by development with a mixture of n-hexane and ethyl acetate. After drying in the air, the spot having the same Rf value as the standard sample of 1-sesamine and giving fluorescence on irradiation with ultraviolet rays was scraped off and subjected to measurement with mass spectrography. In case of the standard sample of 1-sesamine, a parent peak at m/z 354 and peaks of p+1 and p+2 were observed. In case of the specimen as obtained in this Example, a parent peak was observed at m/z 354 or more. The presence of deuterated 1-sesamine in the specimen was thus confirmed.

Example 3

| Material | Amount |
|---|---|
| Deuterated urea | 0.6 g |
| Potassium dihydrogen phosphate | 0.3 g |
| Anhydrous magnesium sulfate | 0.3 g |

The above materials were dissolved in heavy water (1000 ml) and adjusted to pH 6.0 with 1 N NaOD solution in heavy water, followed by filtration through a membrane filter of 0.22 mµ to eliminate contaminating bacteria. In the resultant medium having a deuterium content of about 100 %, a chlorella culture (1 ml) was cultivated at 25°C under irradiation with a light of 5,000 lux while introducing air enriched with 1 % carbon dioxade therein over a period of 48 hours. The culture mixture was centrifuged, and the collected material was washed with water and dried to give a dried chlorella product (about 4 g). The dried chlorella product (about 1 g) was immersed in anhydrous acetone for 24 hours, and acetone was distilled off to give an acetone extract (about 250 mg). The acetone extract was dissolved in a small amount of ether and, after removal of insoluble materials, spotted on a thin layer of silica gel, followed by development with a mixture of n-hexane and ethyl acetate. After drying in the air, the spot having the same Rf value as the standard sample of tocopherol was scraped off and subjected to measurement with mass spectrography. In case of the standard sample of tocopherol (vitamin E) ($C_{29}H_{50}O_2$), a parent peak at m/z 430 was observed. In case of the specimen as obtained in this Example, a parent peak was observed at m/z 480, and any other parent peak was not materially observed between m/z 430 and m/z 479.

0206691

CLAIMS

1. A medium for plant cell culture, wherein not less than 85% of the total hydrogen atoms are deuterium.

2. A medium according to claim 1, which has been made free from contaminating bacteria by filtration and/or irradiation with gamma-rays.

3. A method for the cultivation of plant cells in a medium according to claim 1 or claim 2.

4. A method for producing a deuterated substance, which comprises cultivating plant tissue in a medium according to claim 1 or claim 2, and recovering the deuterated substance from the culture.

5. A method according to claim 3 or claim 4, wherein the cultivation is conducted under irradiation with light.

6. A method according to any of claims 3 to 5, wherein the plant cells are photo-autotrophic.

7. A method according to any of claims 3 to 6, wherein the deuterium content in the medium is gradually increased during cultivation, whereby the plant cells adapt to the conditions of the cultivation.